# FASCICULE DE BREVET EUROPEEN

(11) **EP 3 854 660 B1**
(45) Date de publication et mention de la délivrance du brevet: **02.11.2022**
(21) Numéro de dépôt: 20208753.2
(22) Date de dépôt: 19.11.2020
(51) Int. Cl.: B62B 3/10, A61M 16/00, A61G 12/00, F17C 13/08, A61B 50/13

(54) **CHARIOT HOSPITALIER DE TRANSPORT DE BOUTEILLE DE GAZ AVEC ARBRE DE DISTRIBUTION DE GAZ**
KRANKENHAUSWAGEN FÜR DEN TRANSPORT VON GASFLASCHEN MIT GASVERTEILUNGSSCHACHT
HOSPITAL TROLLEY FOR TRANSPORTING GAS CYLINDER WITH SHAFT FOR DISTRIBUTING GAS

(30) Priorité: 23.01.2020 FR 2000629
(43) Date de publication de la demande: 28.07.2021
(73) Titulaire: L'Air Liquide, société anonyme pour l'Étude et l'Exploitation des procédés Georges Claude, 75007 Paris (FR); Air Liquide Santé France, 75007 Paris (FR)
(72) Inventeur: THEVENET, Louise, 94250 Gentilly (FR); QUENEDEY, Jean-Patrice, 78350 Jouy en Josas (FR); CHANCEL, Fabien, 78350 Jouy en Josas (FR)
(74) Mandataire: Air Liquide

(56) Documents cités:
- EP-A1- 3 854 368
- WO-A1-02/28461
- WO-A1-2006/106328
- CN-U- 208 003 043
- CN-Y- 2 724 679
- FR-A1- 2 926 728

## Description

L'invention porte sur un chariot de transport de bouteille(s) de gaz permettant de transporter une ou plusieurs bouteilles de gaz d'un endroit à un autre au sein d'un bâtiment hospitalier par exemple, c'est-à-dire un chariot hospitalier, comprenant un arbre de distribution de gaz, i.e. de fourniture de gaz respiratoire et de préférence de récupération de gaz expiré par le patient.

En milieu médical, par exemple en cabinet dentaire ou au sein d'une clinique ou d'un hôpital, il est courant d'utiliser des gaz médicaux aux patients, par exemple des gaz analgésiques, tel le N₂O, des gaz d'assistance respiratoire, tel l'oxygène ou l'air médical, ou autres. Ces gaz peuvent être acheminés par un réseau de canalisations ou conditionnés en bouteilles de gaz, comme décrit par EP-A-629812.

Les bouteilles de gaz doivent être transportées par le personnel soignant, tel que médecins, infirmières ou autres, jusqu'à l'endroit où les soins doivent être effectués, par exemple une salle de soins ou une chambre. Pour ce faire, on utilise un chariot de transport de bouteille qui comprend souvent un plateau portant un ou des appareils, matériels ou instruments médicaux, ou des objets ou autres servant pendant les soins, tels que masques, filtre antibactérien, produits désinfectants, papiers, stylos... La (ou les) bouteille est raccordée fluidiquement à un système de distribution de gaz comprenant classiquement un ou plusieurs conduits, un ou des connecteurs etc... Ainsi, les documents EP-A-2574361, EP-A-1977712, FR-A-2926728 , CN2724679Y, CN208003043U et FR-A-2910362 donnent des exemples de tels chariots de transport de bouteilles de gaz.

Afin de distribuer le gaz aux patients, on utilise en général un tuyau ou conduit flexible venant se raccorder par, son extrémité amont, au système de distribution de fluide du chariot, par exemple via un connecteur adapté, alors que son extrémité aval alimente une interface respiratoire servant à fournir le gaz au patient, tel qu'un masque respiratoire ou analogue.

Or, les systèmes de distribution de fluide du chariot sont souvent peu pratiques et peu ergonomiques, ce qui pose des problèmes de raccordement du conduit flexible.

Par ailleurs, les molécules de certains gaz, comme le protoxyde d'azote (N₂O), ne sont pas métabolisées lorsqu'elles sont inhalées par les patients et se retrouvent donc dans les gaz expirés. Il est alors obligatoire de les récupérer et de les retraiter car sinon elles s'accumuleraient dans l'air ambiant des salles des soins ou analogues, ce qui engendrerait d'évidents problèmes de santé pour les personnels soignants par exemple, et par ailleurs des problèmes environnementaux car ces gaz se retrouveraient alors dans l'atmosphère. Pour ce faire, il a déjà été proposé des dispositifs de récupération et/ou de destruction de ces gaz, comme enseignés par EP-A-2247333, WO-A-2009095611 et EP-A-2247332. Toutefois, ces dispositifs de récupération et/ou de destruction de ces gaz ne sont en général pas intégrés aux chariots mais constituent des appareils indépendants. Ces dispositifs doivent donc être amenés sur site en même temps que le chariot de transport des bouteilles de gaz servant à leur administration.

On connait par ailleurs WO2006/106328 qui enseigne un système d'administration de gaz thérapeutique et récupération des gaz expirés par un patient à conduit unique se ramifiant. Là encore, ce système n'est pas intégré à un chariot de transport.

On comprend que ceci n'est pas pratique pour le personnel soignant et par ailleurs augmente l'encombrement général.

Le problème est alors de proposer un chariot de transport d'une ou plusieurs bouteilles de gaz amélioré, lequel ne pose pas les problèmes et inconvénient susmentionnés, en particulier qui soit conçu pour permettre des fourniture et récupération aisées et pratiques de gaz, sans impacter négativement l'encombrement.

La solution concerne alors un chariot de transport de bouteille(s) de gaz mobile comprenant :
- une carcasse délimitant un espace interne dimensionné pour recevoir une ou plusieurs bouteilles de gaz,
- plusieurs roues pour déplacer le chariot sur le sol et
- un système de distribution de gaz relié à au moins une bouteille de gaz et configuré pour acheminer du gaz, le système de distribution de gaz comprend un arbre de distribution de gaz se projetant vers le haut au-dessus du chariot, ledit arbre de distribution de gaz comprenant un tronc et une première branche portée par le tronc, le tronc et la première branche comprenant un premier conduit interne reliant fluidiquement au moins une bouteille de gaz à une première extrémité de raccordement.

Selon l'invention, le tronc et la première branche comprennent un second conduit interne reliant fluidiquement la première extrémité de raccordement à un dispositif de traitement du gaz expiré.

Selon le mode de réalisation considéré, le chariot de l'invention peut comprendre l'une ou plusieurs des caractéristiques suivantes :
- l'arbre de distribution de gaz comprend une seconde branche portée par le tronc.
- la deuxième branche comprend un conduit interne d'acheminement de gaz.
- le tronc forme une portion de base commune aux première et deuxième branches.
- les première et deuxième branches surmontent le tronc.
- les première et deuxième branches sont orientées dans des directions opposées par rapport au tronc.
- le tronc et les première et deuxième branches portées par le tronc ont des formes allongées, notamment tubulaires.
- la première branche comprend une première extrémité de raccordement pour le raccordement fluidique de l'extrémité amont d'un conduit flexible.
- la deuxième branche comprend une seconde extrémité de raccordement pour le raccordement fluidique d'un réservoir de gaz déformable, tel un ballon flexible.
- le tronc et les première et deuxième branches forment une structure ayant une forme générale en Y, c'est-à-dire en Y ou une forme similaire par exemple en V ou en U.
- le dispositif de traitement du gaz expiré comprend un réservoir de stockage de gaz expiré au sein duquel tout ou partie du gaz expiré est stocké/conservé.
- le dispositif de traitement du gaz expiré comprend une unité de catalyse ou d'adsorption de gaz permettant d'éliminer une ou plusieurs molécules, tel le N₂O, se trouvant dans le gaz expiré.
- le dispositif de traitement du gaz expiré est avantageusement agencé dans la carcasse du chariot.
- la carcasse comprend en outre une source d'énergie électrique, telle une batterie rechargeable ou analogue, alimentant notamment le dispositif de traitement du gaz expiré.
- alternativement, la carcasse comprend en outre des moyens de raccordement à un dispositif de traitement du gaz expiré situé à l'extérieur de la carcasse du chariot et lequel vient s'y raccorder pour récupérer le gaz expiré et le traiter.
- la deuxième branche comprend un troisième conduit interne reliant fluidiquement le réservoir de gaz déformable à l'extrémité de la première extrémité de raccordement.
- le conduit flexible comprend l'extrémité amont, une extrémité aval raccordée fluidiquement à une interface respiratoire et une partie intermédiaire située entre lesdites extrémité amont et aval.
- le conduit flexible comprend un premier passage de gaz pour acheminer le gaz respiratoire en direction de l'interface respiratoire, c'est-à-dire le gaz respiratoire inhalé par le patient pendant ses phases inspiratoires.
- le conduit flexible comprend un second passage de gaz pour acheminer le gaz expiré ayant été récupéré par l'interface respiratoire, c'est-à-dire le gaz expiré par le patient pendant ses phases expiratoires.
- les premier et second passages de gaz sont coaxiaux l'un à l'autre.
- la carcasse comprend une paroi de toit surmontant l'espace interne recevant la ou les bouteilles.
- la paroi de toit comprend une ouverture supérieure en communication avec au moins une partie dudit espace interne, au moins une partie dudit conduit flexible pouvant venir se loger dans l'espace interne au travers de l'ouverture supérieure.
- l'arbre de distribution de gaz est situé à l'extérieur de la carcasse.
- l'arbre de distribution de gaz est au moins formé partiellement de polymère, par exemple son revêtement extérieur.
- le système de distribution de gaz vient se raccorder fluidiquement à la ou aux bouteilles de gaz.
- le système de distribution de gaz comprend une ou des conduites de gaz, un ou des connecteurs, un ou des détendeurs de gaz, une ou des vannes, etc....
- un manomètre est agencé sur la première branche de l'arbre de distribution de gaz.
- alternativement, un dispositif indicateur d'autonomie (i.e. temps d'utilisation ou niveau/quantité de gaz résiduel) est agencé sur la première branche de l'arbre de distribution de gaz.
- optionnellement, un organe de réglage de débit est agencé sur la première de l'arbre de distribution de gaz et/ou sur la deuxième branche, par exemple un bouton rotatif.
- un compartiment de rangement est agencé dans l'espace interne de la carcasse en-dessous de l'ouverture supérieure, ladite ouverture supérieure débouchant dans ledit compartiment de rangement de manière à ce qu'au moins une partie intermédiaire du conduit flexible vienne se loger dans ledit compartiment de rangement.
- le compartiment de rangement comprend une paroi périphérique souple, de préférence le compartiment de rangement a une forme de sac.
- le compartiment de rangement comprend des moyens de fixation permettant de le fixer à la carcasse, de préférence à la paroi de toit.
- le conduit flexible comprend une extrémité amont, une extrémité aval à raccorder fluidiquement à une interface respiratoire et une partie intermédiaire située entre lesdites extrémités amont et aval.
- l'extrémité amont et l'extrémité aval sont situées hors de la carcasse lorsque la partie intermédiaire du conduit flexible est logée dans le compartiment de rangement, c'est-à-dire lorsqu'au moins une partie du conduit flexible est rangée dans le compartiment de rangement.
- un dispositif de guidage est agencé au niveau de l'ouverture supérieure pour guider le conduit flexible lorsqu'il est introduit ou extrait du compartiment de rangement au travers de l'ouverture supérieure, en particulier lorsque le conduit flexible coulisse vers l'intérieur ou l'extérieur dudit compartiment de rangement.
- le dispositif de guidage a une forme générale de diabolo comprenant un passage central logeant la partie intermédiaire du conduit flexible.
- le conduit flexible comprend une extrémité aval raccordée fluidiquement à une interface respiratoire, en particulier un masque respiratoire ou analogue, par exemple un masque nasal ou facial (i.e. bucco-nasal).
- le conduit flexible comprend une extrémité aval raccordée fluidiquement à une interface respiratoire par l'intermédiaire d'au moins un élément de raccordement fluidique, tel un (ou des) connecteur ou analogue.
- le conduit flexible a une longueur comprise entre 1 et 10 m, de préférence entre 2 et 5 m.
- l'espace interne de la carcasse est dimensionné pour loger en outre une ou plusieurs bouteilles de gaz, typiquement 1 ou 2 bouteilles de gaz.
- la paroi de toit comprend une ouverture supérieure de forme circulaire, ovale, polygonale, par exemple carrée ou rectangulaire, ou toute autre forme.
- la carcasse comprend des parois périphériques latérales, de préférence 4 parois latérales.
- la carcasse comprend une paroi de dessous ou de fond.
- les parois périphériques latérales se projettent verticalement entre la paroi de fond et la paroi de toit, i.e. de dessus.
- l'espace interne est compris à l'intérieur et/ou délimité par lesdites parois périphériques latérales, paroi de toit et paroi de fond de la carcasse.
- les parois périphériques latérales sont solidaires les unes des autres.
- la surface externe de la paroi de toit est conformée extérieurement en un plateau de soins pouvant recevoir des matériels, des instruments, des appareils, des dispositifs ou autres, utilisés pendant les soins d'un patient.
- il comprend 4 roues pour déplacer le chariot sur le sol.
- il comprend une ouverture latérale agencée dans l'une des parois périphériques latérales et donnant accès à l'espace interne.
- il comprend un tiroir basculant configuré et dimensionné pour recevoir au moins une bouteille de gaz, de préférence deux bouteilles de gaz.
- le tiroir basculant est agencé au niveau de (e.g. dans) l'ouverture latérale et pivotant par rapport à la base de l'ouverture latérale de la carcasse.
- la paroi externe du tiroir est dimensionnée pour obturer complètement l'ouverture latérale, c'est-à-dire que ses dimensions sont telles qu'elles recouvrent toute ou quasiment toute la surface de l'ouverture latérale aménagée dans la paroi périphérique de la carcasse.
- le tiroir basculant comprend un fond de tiroir sur lequel repose la ou les bouteilles de gaz.
- selon un autre mode de réalisation, il comprend une porte pivotante ou coulissante agencée au niveau de l'ouverture latérale de la carcasse de manière à obturer complètement l'ouverture latérale, c'est-à-dire que ses dimensions sont telles que la porte recouvre toute ou quasiment toute la surface de l'ouverture latérale aménagée dans la paroi périphérique de la carcasse.
- la paroi externe du tiroir ou de la porte pivotante ou coulissante comprend des moyens de préhension permettant à l'utilisateur de la saisir manuellement pour opérer une ouverture ou une fermeture du tiroir ou de la porte, par exemple une découpe ou une poignée qui est conçue pour pouvoir être saisie à une main par l'utilisateur.
- la ou les bouteilles de gaz contiennent un gaz médical, i.e. gaz ou mélange gazeux.
- la ou les bouteilles de gaz contiennent un gaz ou mélange gazeux choisi parmi l'oxygène, l'air, un mélange N₂O/O₂ ou autre.
- la ou les bouteilles de gaz ont un corps de forme générale cylindrique.
- la ou les bouteilles de gaz sont équipées d'un robinet de distribution de gaz, avec ou sans système de détente de gaz intégré, c'est-à-dire de type robinet simple ou type robinet à détendeur intégré ou RDI.
- la ou les bouteilles de gaz contiennent du gaz sous pression, typiquement conditionné à une pression maximale de l'ordre de 250 à 300 bar abs.
- la ou les bouteilles de gaz sont en métal, tel de l'acier ou un alliage d'aluminium, ou en matériaux composites.
- la carcasse a une forme générale approximativement parallélépipédique rectangle.
- les roues sont fixées à un châssis, ledit châssis portant en outre la carcasse.
- alternativement, les roues sont fixées directement à la carcasse du chariot.
- la carcasse et/ou le tiroir sont en métal ou en polymère, ou les deux.
- la carcasse comprend des moyens de contrôle des déplacements, par exemple un arceau ou des poignées, manipulables par l'utilisateur afin de pouvoir pousser et diriger le chariot durant ses déplacements sur le sol.
- la carcasse est formée d'une seule pièce ou de plusieurs sous-unités fixées les unes aux autres.

Un chariot selon l'invention est particulièrement bien adapté à une utilisation pour transporter une ou deux bouteilles de gaz au sein d'un lieu de soins ou un bâtiment hospitalier, du type hôpital, clinique, cabinet dentaire, cabinet médical ou autre.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un chariot hospitalier selon l'invention ; et
Fig. 2 est une vue agrandie de l'arbre de distribution de gaz du chariot hospitalier de la Fig. 1.

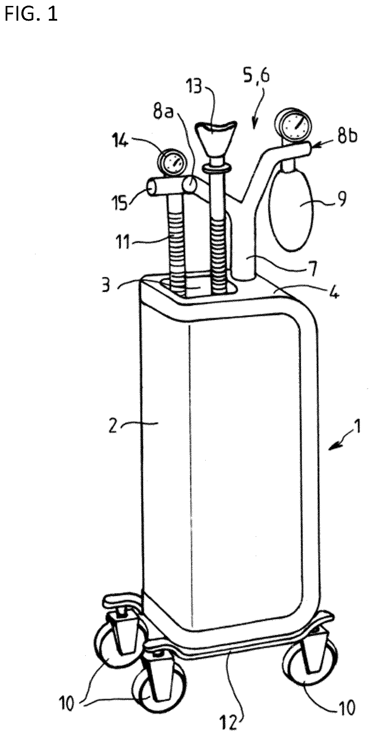

Fig. 1 schématise un mode de réalisation d'un chariot hospitalier 1 de transport de bouteille de gaz selon l'invention. Ce chariot 1 de transport de bouteille de gaz mobile comprend une carcasse 2 délimitant un espace interne recevant une ou plusieurs bouteilles de gaz (non montrées) et éventuellement d'autres compartiments de rangement ou autre. La carcasse 2 comprend par ailleurs une ouverture supérieure 3 agencée dans la paroi de toit 4, et donnant accès à un compartiment de rangement logé dans l'espace interne servant au rangement du tuyau flexible 11.

Afin d'assurer ses déplacements sur le sol au sein d'un bâtiment hospitalier par exemple, sont prévues plusieurs roues 10, par exemple 4 roues, agencées sur un châssis 12 portant en outre la carcasse 2 ou, selon un autre mode de réalisation, directement fixées à la carcasse 2, par exemple à la paroi de fond de la carcasse 2.

Ici, la carcasse 2 comprend 4 parois périphériques latérales, une paroi de dessus ou de toit 4 et une paroi de dessous ou de fond (non visible), fixées ou solidaires les unes des autres, et forment ensemble une structure générale en parallélépipède rectangle. Les 4 parois périphériques latérales se projettent verticalement entre la paroi de fond et la paroi de toit 4, et sont fixées à celles-ci de manière à définir entre elles, l'espace interne de la carcasse 2.

La carcasse 2 peut être formée d'une seule pièce ou de plusieurs sous-unités fixées les unes aux autres. Elle peut être réalisée en métal, en polymère ou les deux.

Afin de permettre l'insertion d'une ou plusieurs bouteilles de gaz dans l'espace interne de la carcasse 2, il est prévu une ouverture latérale (non visible) aménagée dans une des parois périphériques de la carcasse 2, laquelle est fermée par une porte ou un tiroir basculant configuré et dimensionné pour recevoir la ou les bouteilles de gaz, par exemple deux bouteilles agencées en parallèles l'une de l'autre. Chaque bouteille de gaz a une forme générale cylindrique allongée axialement et comprend un robinet ou robinet à détendeur intégré (RDI) servant à contrôler le débit et/ou la pression du gaz délivré. La ou les bouteilles de gaz contiennent avantageusement un gaz médical, par exemple un gaz ou mélange gazeux choisi parmi l'oxygène, l'air, un mélange N₂O/O₂ ou autre.

En outre, il peut aussi être prévu des moyens de contrôle des déplacements du chariot 1, par exemple un arceau ou des poignées, manipulables par l'utilisateur afin de lui permettre de pousser le chariot 1 et de guider ses déplacements sur le sol.

Comme illustré en Fig. 1, le chariot 1 comprend aussi un système de distribution de gaz 5 agencé en partie (i.e. sa portion aval ou aérienne) à l'extérieur et en partie (i.e. sa portion amont) à l'intérieur de la carcasse 2.

La portion amont du système de distribution de gaz 5 est agencée dans la carcasse 2 de manière à venir se raccorder fluidiquement à la ou aux bouteilles de gaz, en particulier à un robinet ou robinet à détendeur intégré (RDI) équipant chaque bouteille, afin de permettre un soutirage et une distribution ensuite du gaz qu'elles contiennent. Ce système de distribution de gaz 5 comprend classiquement un ou des conduites de gaz, connecteurs, vannes, détendeurs, etc....

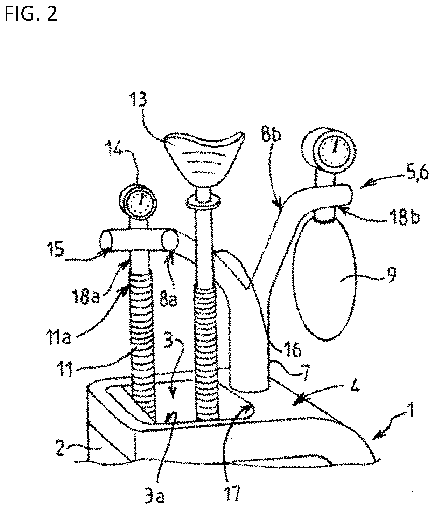

Par ailleurs, selon l'invention, la portion aval du système de distribution de gaz 5 se trouve à l'extérieur de la carcasse et comprend un arbre de distribution de gaz 6 se projetant vers le haut au-dessus du chariot 1, comme visible sur les Fig. 1 et Fig. 2. L'arbre de distribution de gaz 6 est préférentiellement en polymère.

Plus précisément, l'arbre de distribution de gaz 6 comprend un tronc 7 et comprend par ailleurs, dans le mode de réalisation des Fig. 1 et Fig. 2, une première 8a et une deuxième 8b branches portées par le tronc 7 constituant une base commune, c'est-à-dire formant une structure ayant une forme générale approximativement en Y, i.e. en Y, en U, en V ou analogue. Le tronc 7 et les première et deuxième branches 8a, 8b ont des formes allongées, notamment tubulaires. Comme visible sur les Fig. 1 et Fig. 2, le tronc 7 traverse et surmonte la paroi de toit 4.

Toutefois, la deuxième branche 8b est optionnelle. Autrement dit, l'arbre de distribution de gaz 6 pourrait ne comprend que le tronc 7 portant uniquement la première branche 8a. Dans ce mode de réalisation, le tronc 7 et la première branche 8a (i.e. unique branche) pourraient être constitués d'une pièce unique mais aussi de plusieurs éléments raccordés les uns aux autres.

La première branche 8a comprend une première extrémité de raccordement 18a permettant un raccordement fluidique de l'extrémité amont d'un conduit flexible 11a, alors que la deuxième branche 8b comprend une seconde extrémité de raccordement 18b permettant un raccordement fluidique d'un réservoir de gaz déformable 9, tel un ballon flexible.

Dans le mode de réalisation des Fig. 1 et Fig. 2, le tronc 7 et les première et deuxième branches 8a, 8b comprennent des conduits internes d'acheminement de gaz.

Plus précisément, le tronc 7 et la première branche 8a comprennent un premier conduit interne reliant fluidiquement une (ou des) bouteille de gaz à la première extrémité de raccordement 18a portée par la première branche 8a de l'arbre 6, ainsi qu'un second conduit interne reliant fluidiquement la première extrémité de raccordement 18a à un dispositif de traitement du gaz expiré (non visible) situé dans la carcasse 2.

Le premier conduit interne permet donc d'acheminer du gaz respiratoire à faire inhaler au patient depuis une (des) bouteille de gaz jusqu'à la première extrémité de raccordement 18a de la première branche 8a à laquelle est connecté le conduit flexible 11 servant à acheminer ensuite le gaz jusqu'à l'interface respiratoire 13, tel un masque respiratoire ou analogue.

Le second conduit interne permet quant à lui de convoyer le gaz expiré par le patient, qui est récupéré par l'interface respiratoire 13 et acheminé par le conduit flexible 11, jusqu'au dispositif de traitement du gaz expiré situé dans la carcasse 2. Ce dispositif de traitement du gaz expiré permet soit de stocker tout ou partie du gaz expiré par le patient, soit de le purifier en éliminant des molécules de gaz qui s'y trouvent, tel des molécules de N2O, par exemple par adsorption et/ou par catalyse ou un autre système adapté, y compris par perméation.

Pour ce faire, le conduit flexible 11 comprend avantageusement un premier passage de gaz pour acheminer le gaz respiratoire en direction de l'interface respiratoire, c'est-à-dire le gaz respiratoire inhalé par le patient pendant ses phases inspiratoires, et un second passage de gaz pour acheminer le gaz expiré ayant été récupéré par l'interface respiratoire, c'est-à-dire le gaz expiré par le patient pendant ses phases expiratoires.

Préférentiellement, les premier et second passages de gaz sont coaxiaux l'un à l'autre de manière à assurer les flux gazeux, i.e. la circulation du gaz, entre chariot 1 et patient, et inversement, selon la phase respiratoire considérée.

Par ailleurs, la deuxième branche 8b de l'arbre de distribution de gaz 6 comprend un troisième conduit interne reliant fluidiquement le réservoir de gaz déformable 9 à l'extrémité de la première extrémité de raccordement 18a de la première branche 8a.

On peut aussi prévoir un système de basculement agencé dans la carcasse 2 permettant de basculer, de préférence automatiquement, d'une bouteille à une autre pour assurer une continuité de l'approvisionnement en gaz lorsqu'une bouteille est (quasi) vide.

Le conduit flexible 11 comprend une extrémité amont 11a, une extrémité aval 11b à laquelle vient se raccorder fluidiquement, directement ou via un dispositif de connexion 16, tel un connecteur ou autre, l'interface respiratoire 13, tel un masque respiratoire, et une partie intermédiaire située entre lesdites extrémité amont 11a et aval 11b qui vient se loger dans le compartiment de rangement au travers de l'ouverture 3 pratiquée dans le toit 4 de la carcasse 2, comme visible sur la Fig. 2. En effet, lorsque le conduit flexible 11 n'est pas utilisé, par exemple pendant le stockage du chariot ou pendant ses déplacements sur le sol au sein d'un bâtiment, il convient de le ranger afin d'éviter que celui-ci ne se déroule et tombe sur le sol et/ou ne gêne les déplacements du chariot 1.

Typiquement, le conduit flexible 11 peut avoir une longueur comprise entre 1 et 10 m, préférentiellement de 2 à 5 m.

Afin de faciliter et guider l'insertion et l'extraction par coulissement du conduit flexible 11, i.e. de sa partie intermédiaire, dans le compartiment de rangement via l'ouverture supérieure 3, on prévoit préférentiellement un dispositif de guidage 17 qui est agencé au niveau de l'ouverture supérieure 3, par exemple fixé au rebord périphérique 3a de l'ouverture supérieure 3, comme illustré en Fig. 2.

Le dispositif de guidage 17 a par exemple une forme générale de diabolo comprenant un passage central traversé par la partie intermédiaire du conduit flexible 11, c'est-à-dire que la partie intermédiaire du conduit flexible 11 coulisse dans ce passage central du dispositif de guidage 17, lorsque l'utilisateur introduit ou extrait le conduit flexible 11 du compartiment de rangement. De plus, le dispositif de guidage 17 en forme de diabolo peut comprendre deux parois supérieure et inférieure formant des flasques parallèles, i.e. des flasques latérales situées de part et d'autre du corps de diabolo, séparées par une gorge annulaire, et venant prendre en sandwich les surfaces supérieure et inférieure de la paroi de toit 4, au niveau du rebord périphérique 3a de l'ouverture supérieure 3, alors que ledit rebord périphérique 3a de l'ouverture supérieure 3 vient se loger dans la gorge du diabolo.

De préférence, on prévoit aussi un manomètre 14 et un organe de réglage de débit 15, tel un bouton rotatif, qui sont agencés sur la première branche 8a de l'arbre de distribution de gaz 6.

Le manomètre 14 permet de vérifier la pression du gaz acheminé par la première branche 8a, en particulier au sein du premier passage de gaz, alors que l'organe de réglage de débit 15 permet d'ajuster le débit de gaz délivré par la première branche 8a, en particulier le premier passage de gaz.

Un chariot selon l'invention est adapté à une utilisation pour transporter au moins une bouteille de gaz, de préférence une ou des bouteilles de gaz contenant un gaz ou mélange gazeux médical, typiquement un gaz ou mélange gazeux choisi parmi l'oxygène, l'air et un mélange N₂O/O₂ (tel du MEOPA).

## Revendications

1. Chariot (1) de transport de bouteille de gaz mobile comprenant :
- une carcasse (2) délimitant un espace interne dimensionné pour recevoir une ou plusieurs bouteilles de gaz,
- plusieurs roues (10) pour déplacer le chariot sur le sol et
- un système de distribution (5) de gaz relié à au moins une bouteille de gaz, et configuré pour acheminer du gaz, le système de distribution (5) de gaz comprenant un arbre de distribution de gaz (6) se projetant vers le haut au-dessus du chariot (1), ledit arbre de distribution de gaz (6) comprenant un tronc (7) et une première branche (8a) portée par le tronc (7), le tronc (7) et la première branche (8a) comprenant un premier conduit interne reliant fluidiquement au moins une bouteille de gaz à une première extrémité de raccordement (18a),
**caractérisé en ce que** le tronc (7) et la première branche (8a) comprennent un second conduit interne reliant fluidiquement la première extrémité de raccordement (18a) à un dispositif de traitement du gaz expiré.

2. Chariot (1) selon la revendication 1, **caractérisé en ce que** la première branche (8a) comprend une première extrémité de raccordement (18a) pour le raccordement fluidique de l'extrémité amont (11a) d'un conduit flexible (11).

3. Chariot (1) selon la revendication 1, **caractérisé en ce qu'**il comprend en outre une deuxième branche (8b) portée par le tronc (7).

4. Chariot (1) selon la revendication 3, **caractérisé en ce que** la deuxième branche (8b) comprend un conduit interne d'acheminement de gaz.

5. Chariot (1) selon la revendication 3 ou 4, **caractérisé en ce que** la deuxième branche (8b) comprend une seconde extrémité de raccordement (18b) pour le raccordement fluidique d'un réservoir de gaz déformable (9), tel un ballon flexible.

6. Chariot (1) selon la revendication 3, **caractérisé en ce que** le tronc (7) et les première et deuxième branches (8a, 8b) forment une structure ayant une forme en Y.

7. Chariot (1) selon la revendication 3, **caractérisé en ce que** la deuxième branche (8b) comprend un troisième conduit interne reliant fluidiquement le réservoir de gaz déformable (9) à l'extrémité de la première extrémité de raccordement (18a).

8. Chariot (1) selon l'une des revendications 1 ou 3, **caractérisé en ce que** :
- un manomètre (14) ou un dispositif indicateur d'autonomie est agencé sur la première branche (8a) de l'arbre de distribution de gaz (6) et/ou
- un organe de réglage de débit (15) est agencé sur la première branche (8a) de l'arbre de distribution de gaz (6) et/ou sur la deuxième branche (8b), par exemple un bouton rotatif.

9. Chariot (1) selon la revendication 2, **caractérisé en ce qu'**il comprend un conduit flexible (11) raccordé fluidiquement à la première extrémité de raccordement (18a) de la première branche (8a), ledit conduit flexible (11) comprenant :
- un premier passage de gaz pour acheminer le gaz respiratoire en direction d'une interface respiratoire (13)
- un second passage de gaz pour acheminer le gaz expiré ayant été récupéré par l'interface respiratoire (13).

10. Utilisation d'un chariot (1) selon l'une des revendications précédentes pour transporter au moins une bouteille de gaz, de préférence une ou des bouteilles de gaz contenant un gaz ou mélange gazeux choisi parmi l'oxygène, l'air et un mélange N₂O/O₂.

## Patentansprüche

1. Fahrbarer Wagen (1) für den Transport von Gasflaschen, umfassend:
- ein Gehäuse (2), das einen Innenraum begrenzt und dazu ausgelegt ist, eine oder mehrere Gasflaschen aufzunehmen,
- mehrere Räder (10), um den Wagen auf dem Boden zu verfahren, und
- ein Gasverteilungssystem (5), das mit mindestens einer Gasflasche verbunden ist und dazu ausgestaltet ist, Gas weiterzuleiten, wobei das Gasverteilungssystem (5) einen Gasverteilungsbaum (6) umfasst, der sich über dem Wagen (1) nach oben hin erstreckt, wobei der Gasverteilungsbaum (6) einen Stamm (7) und einen von dem Stamm (7) getragenen ersten Zweig (8a) umfasst, wobei der Stamm (7) und der erste Zweig (8a) einen ersten inneren Kanal umfassen, der mindestens eine Gasflasche fluidisch mit einem ersten Anschlussende (18a) verbindet,
**dadurch gekennzeichnet, dass** der Stamm (7) und der erste Zweig (8a) einen zweiten inneren Kanal umfassen, der das erste Anschlussende (18a) fluidisch mit einer Vorrichtung zur Behandlung des ausgeatmeten Gases verbindet.

2. Wagen (1) nach Anspruch 1, **dadurch gekennzeichnet, dass** der erste Zweig (8a) ein erstes Anschlussende (18a) für den fluidischen Anschluss des stromaufwärtigen Endes (11a) eines Schlauchs (11) umfasst.

3. Wagen (1) nach Anspruch 1, **dadurch gekennzeichnet, dass** er ferner einen von dem Stamm (7) getragenen zweiten Zweig (8b) umfasst.

4. Wagen (1) nach Anspruch 3, **dadurch gekennzeichnet, dass** der zweite Zweig (8b) einen inneren Kanal zur Weiterleitung von Gas umfasst.

5. Wagen (1) nach Anspruch 3 oder 4, **dadurch gekennzeichnet, dass** der zweite Zweig (8b) ein zweites Anschlussende (18b) für den fluidischen Anschluss eines verformbaren Gasbehälters (9) wie etwa eines flexiblen Ballons umfasst.

6. Wagen (1) nach Anspruch 3, **dadurch gekennzeichnet, dass** der Stamm (7) und die ersten und zweiten Zweige (8a, 8b) eine Y-förmige Struktur bilden.

7. Wagen (1) nach Anspruch 3, **dadurch gekennzeichnet, dass** der zweite Zweig (8b) einen dritten inneren Kanal umfasst, der den verformbaren Gasbehälter (9) fluidisch mit dem Ende des ersten Anschlussendes (18a) verbindet.

8. Wagen (1) nach einem der Ansprüche 1 oder 3, **dadurch gekennzeichnet, dass**:
- ein Manometer (14) oder eine Reichweitenanzeigevorrichtung an dem ersten Zweig (8a) des Gasverteilungsbaums (6) angeordnet ist und/oder
- ein Volumenstromregelungselement (15) an dem ersten Zweig (8a) des Gasverteilungsbaums (6) und/oder an dem zweiten Zweig (8b) angeordnet ist, zum Beispiel ein Drehknopf.

9. Wagen (1) nach Anspruch 2, **dadurch gekennzeichnet, dass** er einen Schlauch (11) umfasst, der fluidisch an das erste Anschlussende (18a) des ersten Zweigs (8a) angeschlossen ist, wobei der Schlauch (11) umfasst:
- einen ersten Gasdurchlass, um das Atemgas in Richtung einer Beatmungsschnittstelle (13) weiterzuleiten, und
- einen zweiten Gasdurchlass, um das ausgeatmete Gas weiterzuleiten, das von der Beatmungsschnittstelle (13) zurückgeführt wurde.

10. Verwendung eines Wagens (1) nach einem der vorhergehenden Ansprüche zum Transportieren mindestens einer Gasflasche, bevorzugt einer oder mehrerer Gasflaschen, die ein Gas oder Gasgemisch enthalten, das unter Sauerstoff, Luft und einem N₂O/O₂-Gemisch gewählt ist.

## Claims

1. Mobile gas cylinder transport trolley (1) comprising:
- a shell (2) delimiting an internal space dimensioned so as to receive one or more gas cylinders,
- a plurality of wheels (10) for moving the trolley over the ground and
- a gas distribution system (5) connected to at least one gas cylinder, and configured to convey gas, the gas distribution system (5) comprising a gas distribution shaft (6) that projects upwards above the trolley (1), said gas distribution shaft (6) comprising a stem (7) and a first branch (8a) borne by the stem (7), the stem (7) and the first branch (8a) comprising a first internal duct fluidically connecting at least one gas cylinder to a first connection end (18a),
**characterized in that** the stem (7) and the first branch (8a) comprise a second internal duct fluidically connecting the first connection end (18a) to an exhaled gas treatment device.

2. Trolley (1) according to Claim 1, **characterized in that** the first branch (8a) comprises a first connection end (18a) for the fluidic connection of the upstream end (11a) of a flexible duct (11).

3. Trolley (1) according to Claim 1, **characterized in that** it also comprises a second branch (8b) borne by the stem (7).

4. Trolley (1) according to Claim 3, **characterized in that** the second branch (8b) comprises an internal duct for conveying gas.

5. Trolley (1) according to Claim 3 or 4, **characterized in that** the second branch (8b) comprises a second connection end (18b) for the fluidic connection of a deformable gas reservoir (9), such as a flexible balloon.

6. Trolley (1) according to Claim 3, **characterized in that** the stem (7) and the first and second branches (8a, 8b) form a structure having a Y shape.

7. Trolley (1) according to Claim 3, **characterized in that** the second branch (8b) comprises a third internal duct fluidically connecting the deformable gas reservoir (9) to the end of the first connection end (18a).

8. Trolley (1) according to either of Claims 1 and 3, **characterized in that**:
- a manometer (14) or an autonomy indicating device is arranged on the first branch (8a) of the gas distribution shaft (6) and/or
- a flow rate adjustment member (15) is arranged on the first branch (8a) of the gas distribution shaft (6) and/or on the second branch (8b), for example a rotary knob.

9. Trolley (1) according to Claim 2, **characterized in that** it comprises a flexible duct (11) fluidically connected to the first connection end (18a) of the first branch (8a), said flexible duct (11) comprising:
- a first gas passage for conveying the respiratory gas in the direction of a respiratory interface (13) and
- a second gas passage for conveying the exhaled gas that has been recovered by the respiratory interface (13).

10. Use of a trolley (1) according to one of the preceding claims for transporting at least one gas cylinder, preferably one or more gas cylinders containing a gas or gaseous mixture chosen from oxygen, air and an N₂O/O₂ mixture.
